# EUROPEAN PATENT APPLICATION

(11) **EP 1 757 298 A1**
(43) Date of publication of application: **28.02.2007**
(21) Application number: 04821976.0
(22) Date of filing: 16.11.2004
(51) Int. Cl.: A61K 31/7016, A01N 59/00, A01N 61/00, A01N 63/00, A01K 63/04, A01G 7/00, C05F 11/00

(54) **METHOD OF POTENTIATING BIOLOIGCAL ACTIONS OF LIVING ORGANISM BEING IN CONTACT WITH AQUEOUS ENVIRONMENT AND/OR ENLARGING AREA SUITABLE FOR BIOLOGICAL ACTIONS**

(30) Priority: 17.05.2004 JP 2004147045
(71) Applicant: KABUSHIKI KAISHA HAYASHIBARA SEIBUTSU KAGAKU KENKYUJO, Okayama-shi, Okayama 700-0907 (JP)
(72) Inventor: UCHIDA, Yukio, c/o KK Hayashibara Seibutsu Kagaku, Okayama-shi, Okayama 7000907 (JP); OKU, Kazuyuki, c/o KK Hayashibara Seibutsu Kagaku, Okayama-shi, Okayama 7000907 (JP); MIYAKE, Toshio, c/o KK Hayashibara Seibutsu Kagaku, Okayama-shi, Okayama 7000907 (JP)
(74) Representative: Daniels, Jeffrey Nicholas
(86) International application number: PCT/JP2004/016985
(87) International publication number: WO 2005/110432

(57) **Abstract**

The present invention has an object to provide a method for enhancing biological activity and/or expanding suitable region for biological activity of living bodies contacting with aqueous environment, and solves the above object by providing a method for enhancing biological activity and/or expanding suitable region for biological activity of living bodies contacting with aqueous environment by incorporating α,α-trehalose or a saccharide derivative thereof when allowing living bodies to contact with aqueous environment for biological activity of the living bodies contacting with the aqueous environment, and composition and agent for enhancing biological activity and/or expanding suitable region for biological activity of living bodies.

## Description

### TECHNICAL FIELD

The present invention relates to a method for enhancing biological activity and/or expanding suitable region for biological activity of living bodies such as animals and plants contacting with aqueous environment.

### BACKGROUND ART

In general, living bodies contacting with aqueous environment are living as being influenced by mineral concentration as a crucial factor relating to osmotic pressure in aqueous environment. Particularly, mineral ions including sodium, magnesium, potassium and calcium ions are important for biological activity of living bodies. Their concentrations are controlled by ion channels on cell membranes in such a manner of moving inside or outside. Mineral change of aqueous environment often suppresses biological activity or growth of living bodies by accidentally increasing or decreasing the moisture content within their bodies. Examples of such phenomena are salt damage of agricultural products which is caused by typhoons or high-water in seacoast areas or increment of mineral concentration due to evaporation of water in dry land, and growth inhibition of agricultural products which is caused by increased specific metal ions such as aluminum ion and manganese ion in soil pollution areas.

As examples of the method of imparting salt-resistance in living bodies contacting with aqueous environment, those of breeding in the fields of agriculture and fishery or of suppressing mineral stress by introducing salt-resistance genes into living bodies disclosed in Japanese Patent Kokai No. 47,466/2003. Garg A. K., Kim Ju-Kon, Owens T. G., Ranwala A. P., Choi Y., Kochian L. V., and Wu R. J. propose in "Trehalose accumulation in rice plants confers high tolerance levels to different abiotic stresses", Proceedings of the National Academy of Sciences of the United States of America, Vol. 99, No. 25, pp. 15,898-15,903, 2002, a trial of imparting salt-resistance in living bodies by expressing a trehalose producing enzyme in the living bodies to accumulate trehalose therein. However, these methods need vast trials and complicated manipulations in gene recombinant technology, and they are not applicable to natural organisms because they do artificially create new organisms having a salt-resistance. In addition, foods made from genetically-modified organisms as raw materials may not be acceptable in the market by negative public opinions to such foods.

### DISCLOSURE OF THE INVENTION

The present invention has an object to provide a method for enhancing biological activity and/or expanding suitable region for biological activity of living bodies contacting with aqueous environment.

The present inventors have eagerly studied the use of saccharides to attain the above objects. As a result, they unexpectedly revealed that α,α-trehalose and/or saccharide derivatives thereof, incorporated in an aqueous solution as an environment for living bodies, allow living bodies therein to be more active, even at their unsuitable mineral concentrations, i.e., such saccharides are capable of expanding suitable region for biological activity of living bodies in view of mineral concentration, and thus they accomplished the present invention.

The present invention solves the above object by providing a method for enhancing biological activity and/or expanding suitable region for biological activity of living bodies contacting with aqueous environment by coexisting α,α-trehalose or saccharide derivatives thereof with minerals in the aqueous environment, when contacting living bodies with an aqueous mineral solution having a suitable or unsuitable mineral concentration for biological activity of living bodies contacting with the aqueous environment.

The present invention also solves the above object by providing a composition for enhancing biological activity and/or expanding suitable region for biological activity of living bodies.

The present invention also solves the above object by providing an agent for enhancing biological activity and/or expanding suitable region for biological activity of living bodies.

### BEST MODE FOR CARRYING OUT THE INVENTION

The term "method for enhancing biological activity and/or expanding suitable region for biological activity of living bodies contacting with aqueous environment" as referred to as in the present invention usually includes the steps of allowing living bodies to contact with aqueous environment at a part or whole of their bodies and coexisting α,α-trehalose or saccharide derivatives thereof as effective ingredients in the environment.

The α,α-trehalose used in the present invention, a disaccharide composed of two molecules of glucose bound via the α,α-linkage at their reducing ends, is not specifically restricted independently of its purity, form, property or process as long as it enhances biological activity and/or expands suitable region for biological activity. Examples of methods for producing α,α-trehalose include those of allowing a non-reducing saccharide producing enzyme and trehalose releasing enzyme to react with starch or starch hydrolyzate as disclosed by the same applicant as the present invention in Japanese Patent Kokai Nos. 143876/1995, 213283/1995, 298880/1995, 322883/1995, 66187/1996, 66188/1996, 84586/1996 and 336388/1996. These methods provide a remarkably economical advantage that α,α-trehalose can be advantageously produced from low cost starch materials in a high yield. An example of commercialized α,α-trehalose produced by such methods is "TREHA®", a product name of a hydrous crystalline α,α-trehalose commercialized by Hayashibara Shoji Inc. (α,α-trehalose content of 98 w/w % or over, on a dry solid basis). Starch hydrolyzates, by-products due to the method of producing α,α-trehalose from starch materials, are known to be substances easily browned by Mailard reaction with amino groups. If such reducing power affects the present invention, such starch hydrolyzates can be arbitrarily converted into corresponding sugar alcohols with no reducing power by hydrogenation such as Raney nickel method.

The saccharide derivatives of α,α-trehalose used in the present invention can have any binding linkage between α,α-trehalose and additive saccharides by arbitrarily selecting in consideration of desired uses, independently of their purity, form, property and process as long as they have a structure where one or more monosaccharides such as glucose, galactose and fructose are bound to a molecule of α,α-trehalose to enhance biological activity and/or expand suitable region for biological activity of living bodies contacting with aqueous environment. Examples of the saccharide derivatives of α,α-trehalose are α-glycosyl α,α-trehaloses such as α-glucosyl α,α-trehalose, α-maltosyl α,α-trehalose, α-maltotriosyl α,α-trehalose. An example of commercialized such derivatives is "HALLODEX", a product name of a syrup having a solid concentration of 72 w/v % or over and containing about 52 w/w % of α-maltosyl α,α-trehalose, about 4 w/w % of α-glucosyl α,α-trehalose and about 1 w/w % of α-maltotriosyl α,α-trehalose, on a dry solid basis, commercialized by Hayashibara Shoji Inc. An example of commercialized product containing such derivatives and sugar alcohols is "TORNARE", a product name of a syrup having a solid concentration of 72 w/v % or over and containing about 53 w/w % of α-maltosyl α,α-trehalose, about 17 w/w % of maltotetraitol, about 11 w/w % of maltotriitol, about 8 w/w % of maltitol, and about 2 w/w % of sorbitol, on a dry solid basis, commercialized by Hayashibara Shoji Inc.

The term "enhancing biological activity and/or expanding suitable region for biological activity of living bodies contacting with aqueous environment" as referred to as in the present invention means enhancing biological activity of living bodies contacting with aqueous environment having a suitable mineral concentration and further changing an aqueous environment having an unsuitable mineral concentration into an biologically acceptable environment. As a result, undesirable influence on living bodies to be suffered from such aqueous environment is more reduced. Although suitable region for biological activity of living bodies depends on the kind of minerals and species of living bodies, the effect of the present invention, which enhances biological activity and/or expands suitable region for biological activity of living bodies, can be exerted for various minerals. Such effect can be confirmed by examining the degree of enhancing biological activity and/or expanding suitable region for biological activity of living bodies at various concentrations of alkaline metals such as sodium and potassium. For example, in the case of mineral solutions containing sodium as a main ingredient, the most suitable concentration for biological activity may correspond to that of seawater or brackish water. Therefore, the mineral concentration range additionally expands or narrows by ± 2 w/v %, preferably ± 1.5 w/v %, more preferable ± 1 w/v % in number in addition to the percentage of the mineral concentration of seawater or brackish water. The present invention can enhance biological activity and/or expand suitable region for biological activity of living bodies in such ranges. Explaining concretely, in the case that a suitable total mineral concentration in aqueous environment corresponds to that of seawater (3 w/v %), the present invention is effective in the range of 1 to 5 w/v %, preferably 1.5 to 4.5 w/v %, more preferably 2 to 4 w/v %. While, in the case of brackish water whose mineral concentration may change in the range of 0 to 3 w/v %, for example, in the case of living bodies whose suitable concentration is 1.5 w/v %, the present invention is effective in the range of 0 to 3.5 w/v %, preferably 0 to 3.0 w/v %, and more preferably 0.5 to 2.5 w/v %. In the case of fresh water usually containing no mineral, the present invention is effective in the range of 0 to 2 w/v %, preferably 0 to 1.5 w/v %, more preferably 0 to 1 w/v %. In the above cases, α,α-trehalose or saccharide derivatives thereof are allowed to incorporate in aqueous environment in an amount in the range of 0.01 to 4 w/v %, preferably 0.1 to 3 w/v %, on a dry solid basis. In the case of under 0.01 w/v %, the desired effect may not be exerted. In the case of over 4 w/v %, it is not preferable because the effect is not exerted dose-dependently and osmotic pressure may be unexpectedly increased by incorporating α,α-trehalose or saccharide derivatives thereof.

The method for enhancing biological activity and/or expanding suitable region for biological activity of living bodies of the present invention is applied for many kinds of aqueous solutions of minerals by using α,α-trehalose and/or saccharide derivatives thereof as effective ingredients. Examples of such minerals are ion compounds or salts of sodium, magnesium, aluminum, potassium, calcium, chromium, manganese, iron, cobalt, nickel, copper, zinc, molybdenum, lead, etc. The method of the present invention can be applied for a composition containing such minerals and α,α-trehalose and/or saccharide derivatives thereof, which is produced by coexisting α,α-trehalose and/or saccharide derivatives thereof in a mixture of one or more minerals. The composition is advantageously used for enhancing biological activity and/or expanding suitable region for biological activity of living bodies.

α,α-Trehalose and/or saccharide derivatives thereof is (are) preferably contained in the above composition for enhancing biological activity and/or expanding suitable region for biological activity of living bodies in an amount in the range of 0.01 to 4 w/v %, preferably 0.1 to 3 w/v % in case that an aqueous solution contains minerals at a concentration in the range of 0 to 5 w/v %.

In case that the composition is prepared into a pre-mixed powder or pre-mixed concentrated solution, the ratio of α,α-trehalose and/or saccharide derivatives thereof to minerals can be arbitrarily decided in consideration of the kind of minerals and objective living bodies, and is usually 1:100 to 100:1, preferably 1:20 to 20:1, and more preferably 5:1 to 1:5 by weight.

Explaining concretely the uses of the present invention, it is applied for water for agriculture to prevent salt damages even in the case of water containing minerals at a high concentration such as seawater, which is unsuitable for agriculture, by diluting such water, if necessary.

The present invention biologically activates fishes or shellfishes to prevent weight loss (deterioration of flesh property) and accumulate useful *umami* ingredients when used as water for fish preserve or aquarium. In addition, the present invention reduces undesirable influences on breeding of living bodies due to mineral change, or increasing or decreasing of moisture content caused by rainwater or evaporation. When used in seawater such as natural seawater, deep seawater, and artificial seawater, culture medium, cleaning fluid for living bodies, etc., the composition of the present invention enhances biological activity and/or expands suitable region for biological activity of living bodies and reduces undesirable influences to living bodies contacting aqueous environment of aqueous mineral solutions having an unsuitable mineral concentration. In addition, when formulated in the form of a natural seawater, deep seawater, artificial seawater, culture medium, or cleaning fluid in a premixed solid or premixed concentrated solution, the composition is arbitrarily used for valuable animals and plants because it expands the permissible range of measuring error when it is dissolved in or diluted with water. The composition of the present invention also sustains and develops living bodies even when used in an aqueous environment having an unsuitable mineral concentration for biological activity. Therefore, it enables to breed or cultivate freshwater fishes and shellfishes, terrestrial plants, marine fishes and shellfishes, marine plants, etc., even in solutions with an unsuitable mineral concentration. The composition is used for salt bath aiming to treat diseases of freshwater fishes and shellfishes such as gold fishes and *nishikigoi* (colored carp). In such case, the composition is more effective on treatment because it improves hyponatremia of the fishes to protect them from undesirable influences of mineral aqueous solutions with an unsuitable mineral concentration for them. In addition, in this case, since it also reduces undesirable influences on other organisms such as algae and water plants, it enables to treat fishes even in aquariums or ponds. When the composition of the present invention is applied for the above cases, it can be used in combination with medicaments applicable to diseases or symptoms of the fishes, such as antibiotics, bactericides, antibiotics and vitamins. When the method of the present invention for enhancing biological activity and/or expanding suitable region for biological activity of living bodies is applied for cultivating plants, it increases the rate of exchanging minerals absorbed by living bodies into organic minerals. Therefore, it effectively imparts minerals into fresh vegetables such as sprout foods. In addition, when the method of the present invention can be applied for removing mud from living fishes and shellfishes, or deodorizing or transferring living fishes and shellfishes, it more effectively attains the desired objects without loss of biological activity of living bodies.

The living bodies contacting with aqueous environment, which are desirably applicable for the present invention, are illustrated with animals and plants living in seawater, brackish, freshwater or terrarium, concretely general fishes, shellfishes and plants. Examples of such fishes and shellfishes are fishes such as a salmon, trout, *Salvelinus pluvius,* sweet fish, *Hypomesus olidus,* sprat, sardine, eel, sea ell, flying fish, saury, codfish, angler, snook, mullet, sea bream, horse-mackerel, amberjack, mackerel, bonito, sierra, tuna, hairtail, rockfish, rocktrout, stone fish, halibut, turbot, blowfish, threadsail filefish, shark, rocker, carp, cruciam carp, dikkop, loach and catfish; acuarium fishes such as a tropical fish, gold fish, *nishikigoi* (colored carp) and killifish; shrimps and prawns such as tiger shrimp and crawdish; crab such as a swimming crab, horsehair crab, *Eriocheir simensis,* queen crab, king crab and mantis shrimp; shellfishes such as an abalone, turban shellfish, Babylonia, snail, ark shellfish, orange shellfish, heart clam, Chinese Mactra, Gould's Jackknife claim, short-necked clam, clam, oyster, scallop, mussel and corbicula; echinoderm such as a sea urchin and holothurian; molluscan such as a squid and octopus; ascidian; jellyfish; and fertilized egg, ovum, sperm and alevin thereof. Examples of such plants are agricultural products such as rice, wheat, soy bean and corn; algae such as a soft seaweed and kelp; vegetables such as a cabbage and lettuce; sprout foods such as a bean sprout and alfalfa. In the case of applying to plants, the present invention attains an object of the present invention by contacting the whole or a part of the plants such as a leaf, stem, root and petal with aqueous environment.

The following experiments explain the influence of saccharides for enhancing biological activity and/or expanding biological activity of living bodies in detail using a short-necked clam living in seawater, corbicula, Carassium, goldfish living in freshwater as animal examples; and unpolished rice and alfalfa in freshwater as plant examples.

### Experiment 1:

### Selection of saccharides capable of influencing the enhancement of biological activity of short-necked clam

Suitable region for biological activity of short-necked clam (*Ruditapes philippinarum*), as a fish or shellfish living in seawater, was examined by adding various saccharides to mineral aqueous solutions with various concentrations. In detail, solar salt (85% by weight of sodium chloride and 15% by weight of magnesium) as minerals was dissolved in running water kept in a container for two days to give final concentrations described in the following Table 1. Saccharide (any one of D-glucose, maltose, sucrose, maltitol, α,α-trehalose, α-maltosyl α,α-trehalose, maltotetraose and cyclic tetrasaccharide) was dissolved in the resulting salt solutions to give a final concentration of 1 w/v %, on a dry solid basis. Each of the resulting solutions was placed in a plastic bat. Eighteen pre-selected active short-necked clam in one group were placed in the bat. The bat was shaded with a black cloth and left at ambient temperature (18°C) for 16 hours. As a control, an aqueous solar salt solution without any saccharides was prepared. Saccharides except for α-maltosyl α,α-trehalose as a saccharide derivatives thereof and cyclic tetrasaccharide were chosen from commercialized products. α-Maltosyl α,α-trehalose as saccharide derivatives of α,α-trehalose in a powder form with a purity of 98 w/w % or over was prepared by the method disclosed in Experiment 4 in Japanese Patent Kokai No. 143876/1995. Cyclic tetrasaccharide in a crystalline form with a purity of 98 w/w % or over, on a dry solid basis, was prepared by the method disclosed in Example A-7 in International Patent Publication No. WO02/10361.

Influence of various saccharides was judged by observing the degree of extending aqueduct of the shellfish; 3 points: its aqueduct is well extended; 2 points: its aqueduct is slightly extended; 1 point: its aqueduct is not extended but its shells are opened; 0 point: its shells are closed. The effect on biological activity was judged by the total point of 18 shellfishes. The result is shown in Table 1.

**Table 1**

| Saccharide | Judgment score for biological activity in aqueous solutions at various mineral concentrations (w/v)% | | | | | | | | | | | | |
|---|---|---|---|---|---|---|---|---|---|---|---|---|---|
| | 0 | 0.5 | 1.0 | 1.5 | 2.0 | 2.5 | 3.0 | 3.5 | 4.0 | 4.5 | 5.0 | 5.5 | 6.0 |
| None (control) | 5 | 11 | 10 | 14 | 29 | 42 | 48 | 44 | 28 | 12 | 7 | 0 | 0 |
| D-Glucose | 7 | 12 | 12 | 14 | 30 | 41 | 48 | 44 | 31 | 24 | 11 | 4 | 0 |
| Maltose | 4 | 12 | 14 | 15 | 32 | 44 | 48 | 42 | 32 | 25 | 8 | 3 | 0 |
| Sucrose | 7 | 11 | 14 | 16 | 31 | 44 | 48 | 45 | 30 | 21 | 9 | 0 | 0 |
| Maltitol | 8 | 11 | 15 | 20 | 30 | 41 | 48 | 47 | 32 | 16 | 12 | 5 | 0 |
| α,α-Trehalose | 12 | 16 | 38 | 44 | 47 | 51 | 54 | 50 | 50 | 49 | 34 | 10 | 5 |
| α-Maltosylα,α-trehalose | 11 | 14 | 36 | 40 | 45 | 49 | 53 | 50 | 47 | 45 | 33 | 11 | 2 |
| Maltotetraose | 7 | 11 | 12 | 16 | 28 | 45 | 46 | 42 | 31 | 26 | 14 | 5 | 0 |
| Cyclic tetrasaccharide | 6 | 10 | 15 | 16 | 30 | 43 | 49 | 41 | 30 | 17 | 11 | 0 | 0 |

As shown in Table 1, in the case of an aqueous mineral (solar salt) solution with no saccharide as a control, the shellfishes actively extended their aqueducts under the mineral concentration in the range of 2.5 to 3.5 w/v % corresponding to or arround the mineral concentration of seawater (total points 42 to 48). While, the shellfishes were not active in the mineral concentration in the range of 2.0 w/v % or below and 4.0 w/v % or over (total points 29 or 28 or less). These results revealed that the suitable region of mineral concentration for biological activity of the shellfishes was in the range of 2.5 to 3.5 w/v %. While, in the aqueous mineral solutions containing α,α-trehalose or α-maltosyl α,α-trehalose, biological activities of the shellfishes were enhanced at mineral concentrations in the range of 2.5 to 3.5 w/v % corresponding to or around the mineral concentration of seawater (total points 50 to 54, or 49 to 53), and further the shellfishes were active as shown by high scores at the mineral concentrations in the range of 1.0 to 5.0 w/v %, preferably 1.5 to 4.5 w/v %, and more preferably 2.0 to 4 w/v %. The result revealed that α,α-trehalose and α-maltosyl α,α-trehalose enhanced biological activity and/or expanded suitable region for biological activity of the shellfishes, but not other saccharides. Therefore, the result shows that α,α-trehalose and α-maltosyl α,α-trehalose are capable of enhancing biological activity and/or expanding suitable region for biological activity of fishes and shellfishes living in seawater.

### Experiment 2:

### Examination of the concentration of α,α-trehalose or α-maltosyl α,α-trehalose which influences the enhancement of biological activity of short-necked clam

To investigate the effective concentration range of α,α-trehalose or α-maltosyl α,α-trehalose against a mineral solution with a concentration of 1.5 w/v % or 4.5 w/v %, where α,α-trehalose or α-maltosyl α,α-trehalose was remarkably effective in Experiment 1, the following experiment using α,α-trehalose or α-maltosyl α,α-trehalose at a concentration of 0.01, 0.1, 1, 2, 3 or 4 w/v % was carried out according to the method in Experiment 1. As a control, mineral aqueous solutions with a concentration of 1.5 w/v % or 4.5 w/v % without any of the saccharides were prepared and carried out in the same manner. The result is shown in Table 2.

**Table 2**

| Saccharide concentration (w/v%) | Judgment score of biological activity | | | | Note |
|---|---|---|---|---|---|
| | Mineral concentration of 1.5 w/v% | | Mineral concentration of 4.5 w/v% | | |
| | α,α-Trehalose | α-Maltosyl α,α-trehalose | α,α-Trehalose | α-Maltosyl α,α-trehalose | |
| 0 | 14 | | 12 | | Control |
| 0.01 | 19 | 18 | 21 | 16 | The present invention |
| 0.1 | 38 | 35 | 35 | 37 | The present invention |
| 1 | 44 | 41 | 50 | 44 | The present invention |
| 2 | 47 | 43 | 51 | 45 | The present invention |
| 3 | 38 | 34 | 31 | 30 | The present invention |
| 4 | 21 | 16 | 18 | 15 | The present invention |

As shown in Table 2, α,α-trehalose or α-maltosyl α,α-trehalose marked high scores at a concentration in the range of 0.01 to 4 w/v %, preferably 0.1 to 3 w/v % and was effective on expanding suitable region for biological activity of short-necked clam. The results of the above experiment using the shellfish show that α,α-trehalose and α-maltosyl α,α-trehalose are capable of enhancing biological activity and/or expanding suitable region for biological activity of fishes and shellfishes living in seawater, including short-necked clam.

### Experiment 3:

### Influence of saccharides on flesh property and umami ingredients in edible part of short-necked clam

To examine the influence on flesh property and *umami* ingredients of short-necked clam, the following experiment was carried out by incorporating 1 w/v % of trehalose or sucrose in a solution with a salt concentration corresponding to that of usual seawater (3 w/v %).

In detail, according to the method in Experiment 1, solar salt (85% by weight of sodium chloride and 15% by weight of magnesium) as minerals was dissolved in tap water kept in a container for two days to give a final concentration of about 3 w/v %. α,α-Trehalose or sucrose was dissolved in the resulting salt solutions to give a final concentration of 1 w/v %, on a dry solid basis. The resulting each solution was placed in a plastic bat. Six pre-selected active short-necked claims in one group were placed in the bat. The bat was shaded with a black cloth and left at ambient temperature (18°C) for 16 hours. As a control, an aqueous solar salt solution without any saccharides was prepared. The edible parts were taken off the broken shells and pushed by fingers to evaluate the elasticity in comparison with that of the control. The edible parts of the shellfishes were admixed with 50 ml of 20 mM sulfuric acid and homogenized with a homogenizer. The resulting mixtures were centrifuged to collect aqueous solutions. The resulting solutions were filtrated with "ULTRAFILTER UNIT USY-1", a product name of a filter commercialized by ADVANTECH Corporation, to remove contaminant proteins. The resulting solutions were subjected to "IC500P", a product name of an ion chromatoanalyzer commercialized by Yokogawa Electric Corporation, equipped with analyzing columns "SCS5-252" and "PCS5-052" (commercialized by Yokogawa Electric Corporation) in a usual manner to quantitatively analyze various organic acids (succinic acid, malic acid, acetic acid, propionic acid, and total such acids) as *umami* ingredients and evaluate relative values to the amount of various organic acids in the edible part of the control. The result was shown in Table 3.

**Table 3**

| Saccharide | Flesh property (Elasticity) | Content of organic acid (Relative value) | | | | |
|---|---|---|---|---|---|---|
| | | Succinic acid | Malic acid | Acetic acid | Propionic acid | Total acids |
| α,α-Trehalose | Satisfactory | 167% | 181% | 120% | 122% | 163% |
| Sucrose | Unchanged | 111% | 127% | 98% | 137% | 114% |

As shown in Table 3, it was confirmed that the edible parts treated with the solution containing α,α-trehalose had an elastic flesh property, and were active without loss of weight. The content of organic acids as *umami* ingredients was increased, particularly the contents of succinic acid and malic acid were increased. The result reveals that α,α-trehalose is not only assimilated by short-necked clam for energy source to enhance its biological activity but it also imparts satisfactory elastic flesh property to the edible parts and increases the content of *umami* ingredients.

### Experiment 4:

### Screening of saccharide influencing the enhancement of biological activity of corbicula

Suitable region for biological activity was examined by using colbicula (*Corbicula leana*) as fishes and shellfishes living in fresh water in the case of enhancing their biological activity by using aqueous mineral solutions containing various saccharides. In detail, solar salt (85% by weight of sodium chloride and 15% by weight of magnesium) as minerals was dissolved in tap water kept in a container for two days to give final concentrations described in the following Table 4. Saccharide (any one of D-glucose, maltose, sucrose, maltitol, α,α-trehalose, α-maltosyl α,α-trehalose, maltotetraose and cyclic tetrasaccharide) was dissolved in the resulting salt solutions to give a final concentration of 1 w/v %, on a dry solid basis. The resulting each solution was placed in a plastic bat. Eighteen pre-selected active shellfishes in one group were placed in the bat. The bat was shaded with a black cloth and left at ambient temperature (18°C) for 16 hours. As a control, an aqueous solar salt solution without saccharides was prepared. Biological activity of the shellfish was judged by measuring extend their aqueducts macroscopically in the same manner as in Experiment 1. The result is shown in Table 4.

**Table 4**

| Saccharide | Judgment score of biological activity in mineral solutions at various concentrations | | | | | | |
|---|---|---|---|---|---|---|---|
| | 0 | 0.5 | 1.0 | 1.5 | 2.0 | 2.5 | 3.0 |
| None (Control) | 50 | 48 | 21 | 15 | 10 | 2 | 0 |
| D-glucose | 50 | 51 | 25 | 18 | 15 | 5 | 0 |
| Maltose | 51 | 49 | 18 | 15 | 14 | 9 | 0 |
| Sucrose | 51 | 46 | 21 | 11 | 8 | 7 | 0 |
| Maltitol | 50 | 47 | 25 | 18 | 13 | 2 | 0 |
| α,α-Trehalose | 54 | 53 | 52 | 49 | 42 | 20 | 6 |
| α-Maltosylα,α-trehalose | 54 | 53 | 45 | 44 | 37 | 24 | 4 |
| Maltotetraose | 51 | 49 | 19 | 14 | 11 | 9 | 0 |
| Cyclic tetrasaccharide | 51 | 50 | 29 | 16 | 13 | 12 | 1 |

As shown in Table 4, in the case of aqueous mineral (solar salt) solution without any saccharides, corbicula was active at the mineral concentrations in the range of 0 to 0.5 w/v % (score 50 to 48 points). Whereas, they were not active at mineral concentrations in the range of 1 w/v % or more (score 21 points or less) to be considered as unsuitable for biological activity. While, the aqueous mineral solutions containing α,α-trehalose or α-maltosyl α,α-trehalose activated the shellfish at mineral concemtrations in the range of 0 to 0.5 w/v % (score 54 to 53), in addition, such solutions activated them in the range of 0 to 2.0 w/v %, preferably 0 to 1.5 w/v %, 0 to 1 w/v % as high scores. α,α-Trehalose or α-maltosyl α,α-trehalose reduced undesirable influences from the aqueous mineral solutions. The other saccharides did not show such result. The fact indicates that α,α-trehalose and α-maltosyl α,α-trehalose enhance biological activity of freshwater fishes and shellfishes and/or expand suitable region for biological activity when incorporated in aqueous environment.

### Experiment 5

### Study for the influence of concentration of the coexisting α,α-trehalose or α-maltosyl α,α-trehalose on enhancing biological activity of corbicula

Effective concentration range of α,α-trehalose or α-maltosyl α,α-trehalose on enhancing biological activity and/or influencing on suitable region for biological activity of colbicula (*Corbicula leana*) was determined according to the method of Experiment 4 by examining biological activity of the shellfish in the aqueous mineral solution with a mineral concentration of 1.5 w/v %, where α,α-trehalose or α-maltosyl α,α-trehalose was effective in Experiment 4, containing α,α-trehalose or α-maltosyl α,α-trehalose at a concentration of 0.01, 0.1, 1, 2, 3 or 4 w/v %, on a dry solid basis. As a control, a solution containing 1.5 w/v % of mineral without such saccharides was prepared and treated in the same manner. The result is shown in Table 5.

**Table 5**

| Saccharide Concentration (w/v%) | Judgment score of biological activity | | Note |
|---|---|---|---|
| | α,α-Trehalose | α-Maltosyl α,α-trehalose | |
| 0 | 15 | | Control |
| 0.01 | 21 | 19 | The present invention |
| 0.1 | 39 | 35 | The present invention |
| 1 | 52 | 45 | The present invention |
| 2 | 48 | 41 | The present invention |
| 3 | 39 | 34 | The present invention |
| 4 | 25 | 21 | The present invention |

As shown in table 5, α,α-trehalose or α-maltosyl α,α-trehalose, showed high scores at the concentrations in the range of 0.01 to 4 w/v %, preferably 0.1 to 3 w/v % in the aqueous mineral solution with a mineral concentration of 1.5 w/v %, was effective on enhancing biological activity and/or expanding suitable region for biological activity of the shellfishes. The above experiments using the shellfish reveals that α,α-trehalose and α-maltosyl α,α-trehalose are capable of enhancing biological activity and/or expanding suitable region for biological activity for seawater fishes or shellfishes, including corbicula.

### Experiment 6

### Influence of the coexistence of α,α-trehalose and α-maltosyl α,α-trehalose in an aqueous mineral solution on the germination of unpolished rice

Suitable mineral range of aqueous mineral solutions containing saccharides was examined by using unpolished rice as an agricultural product. In detail, a commercialized purified salt as a mineral was dissolved in tap water to give a final concentration of 0.5, 1.0, 1.5, or 2.0 as described in the following Table 6. α,α-Trehalose or α-maltosyl α,α-trehalose was dissolved in the resulting solutions to give a final concentration of 1 w/v %, on a dry solid basis. Absorbent cotton was allowed to absorb the resulting solution in an appropriate amount and placed in a plastic laboratory dish with a cover. Commercialized unpolished rice grains were placed on the cotton in the dish by 20 grains per group and kept at ambient temperature (25°C) under a light-shaded condition by covering with a black cloth. As controls, an aqueous mineral solution without saccharides, and water without saccharides and minerals were prepared. After 24, 48, 72, 96 and 120 hours, the unpolished rice grains were observed to examine the progress of germination and count the number of germinated rice grains. The result is shown in Table 6.

**Table 6**

| Mineral Concentration (w/v%) | Saccharide | Germinated grains (Number) | | | | |
|---|---|---|---|---|---|---|
| | | 24 hours | 48 hours | 72 hours | 96 hours | 120 hours |
| 0 | None | 0 | 20 | 20 | 20 | 20 |
| 0.5 | None | 0 | 9 | 14 | 20 | 20 |
| | α,α-Trehalose | 0 | 20 | 20 | 20 | 20 |
| | α-Maltosyl α,α-trehalose | 0 | 15 | 19 | 20 | 20 |
| 1.0 | None | 0 | 0 | 4 | 13 | 15 |
| | α,α-Trehalose | 0 | 17 | 20 | 20 | 20 |
| | α-Maltosyl α,α-trehalose | 0 | 3 | 10 | 16 | 16 |
| 1.5 | None | 0 | 0 | 0 | 2 | 2 |
| | α,α-Trehalose | 0 | 1 | 4 | 6 | 6 |
| | α-Maltosyl α,α-trehalose | 0 | 1 | 3 | 5 | 5 |
| 2.0 | None | 0 | 0 | 0 | 0 | 0 |
| | α,α-Trehalose | 0 | 0 | 0 | 0 | 0 |
| | α-Maltosyl α,α-trehalose | 0 | 0 | 0 | 0 | 0 |

As shown in Table 6, the numbers of germinated unpolished rice grains after 48 to 120 hours in the aqueous mineral solutions containing α,α-trehalose or α-maltosyl α,α-trehalose were greater than those in the aqueous mineral solutions. Referring to the result after 72 hours, the number of germinated rice grains in the case of using water without both of saccharides and minerals was 20. While, the numbers of germinated rice grains in the case of using aqueous mineral solutions with mineral concentrations of 0.5 w/v %, 1.0 w/v %, 1.5 w/v % and 2.0 w/v % were respectively 14, 4, 0 and 0. In contrast, the numbers of germinated rice grains in the case of using aqueous mineral solutions containing α,α-trehalose or α-maltosyl α,α-trehalose were respectively 20, 20, 4 and 0, or 19, 10, 3 and 0. As a result, incorporating α,α-trehalose or α-maltosyl α,α-trehalose enhanced the germination of unpolished rice grains in aqueous mineral solutions. The result revealed that the coexistence of α,α-trehalose or α-maltosyl α,α-trehalose was effective on the germination of unpolished rice grains in aqueous mineral solutions with mineral concentrations of 1.5 w/v % or less, preferably 1.0 w/v % or less. Therefore, α,α-trehalose or α-maltosyl α,α-trehalose effectively expands suitable region for biological activity of unpolished rice grains in aqueous mineral solutions.

### Experiment 7

### Effective concentration of α,α-trehalose or derivatives thereof on the germination of unpolished rice grains in an aqueous mineral solution

Effective concentration of α,α-trehalose or α-maltosyl α,α-trehalose on expanding suitable region for biological activity of unpolished rice grains were determined by examining the number of germinated unpolished rice grains and germination time as an index in accordance with the method in Experiment 6 in the aqueous mineral solution with mineral concentration of 1.5 w/v %, where α,α-trehalose or α-maltosyl α,α-trehalose was effective in Experiment 6, containing α,α-trehalose or α-maltosyl α,α-trehalose in a concentration of 0.01, 0.1, 1, 2, 3, 4 or 6 w/v %, on a dry solid basis. As a control, water without saccharides or minerals was used. The result is shown in Table 7.

**Table 7**

| Saccharide | Concentration (w/v%) | Germinated grains (Number) | | | |
|---|---|---|---|---|---|
| | | 48 hours | 72 hours | 96 hours | 120 hours |
| None (Control) | 0 | 0 | 0 | 2 | 2 |
| α,α-Trehalose | 0.01 | 0 | 1 | 2 | 3 |
| | 0.1 | 0 | 2 | 5 | 6 |
| | 1 | 1 | 4 | 6 | 6 |
| | 2 | 3 | 6 | 7 | 7 |
| | 3 | 3 | 6 | 6 | 6 |
| | 4 | 1 | 6 | 6 | 6 |
| | 6 | 0 | 2 | 2 | 2 |
| α-Maltosyl α,α-trehalose | 0.01 | 0 | 2 | 2 | 2 |
| | 0.1 | 0 | 2 | 4 | 5 |
| | 1 | 1 | 3 | 5 | 5 |
| | 2 | 1 | 6 | 6 | 6 |
| | 3 | 1 | 4 | 5 | 5 |
| | 4 | 0 | 3 | 3 | 3 |
| | 6 | 0 | 0 | 1 | 1 |

As shown in Table 7, α,α-trehalose or α-maltosyl α,α-trehalose exerted the effects of enhancing biological activity and/or expanding suitable region for biological activity of unpolished rice grains in a 1.5 w/v % aqueous mineral solution with mineral concentration in the range of 0.01 to 4 w/v %, preferably 0.1 to 3 w/v %. The above experimental results using unpolished rice reveal that α,α-trehalose and α-maltosyl α,α-trehalose are capable of enhancing biological activity and/or expanding suitable region for biological activity of agricultural products including unpolished rice.

### Experiment 8

### Influence of α,α-trehalose or α-maltosyl α,α-trehalose on the germination of sprout foods (alfalfa) in an aqueous mineral solution

Absorbent cotton was allowed to absorb aqueous solution containing on a dry solid basis 1 w/v % of mineral (sodium chloride) and 1 w/v % of α,α-trehalose or α-maltosyl α,α-trehalose and placed in a transparent container. One hundred per group commercialized alfalfa seeds were placed in the container. As references, waters containing either of mineral, α,α-trehalose or α-maltosyl α,α-trehalose were used. As a control, water was used. After covered with a transparent lid, the container was placed in a room under a borrowed light at 18°C for three days to cultivate alfalfa. The grown alfalfa plants were measured to calculate average value of aerial part (stem) height. Relative extension rate was calculated in such a manner of comparing the average value of each sample with that of control. In addition, 20 panels were judged in view of flavor and taste in such a manner of comparing each sample with control cultivated in water. The result is shown in Table 8.

**Table 8**

| Sample | Relative extension (%) | Judgment of flavor and taste | | | | Note |
|---|---|---|---|---|---|---|
| | | Good | Slightly good | Normal | Bad | |
| 1 (w/v)% mineral | 28 | 2 | 6 | 11 | 1 | Reference |
| 1 (w/v)% α,α-trehalose | 101 | 1 | 12 | 7 | 0 | Reference |
| 1 (w/v)% α-maltosyl α,α-Trehalose | 105 | 1 | 13 | 6 | 0 | Reference |
| 1 (w/v)% mineral + 1 (w/v)% α,α-trehalose | 96 | 13 | 5 | 2 | 0 | The present invention |
| 1 (w/v)% mineral + 1 (w/v)% α-maltosyl α,α-trehalose | 97 | 14 | 5 | 1 | 0 | The present invention |

As the result from Table 8, alfalfa cultivated with the water containing only mineral (sodium chloride) showed lower relative extension of aerial part and was judged that its flavor and taste were only slightly improved. In contrast, when cultivated with water containing α,α-trehalose or α-maltosyl α,α-trehalose, alfalfa kept a normal extension rate for aerial part and improved in flavor and taste in a certain degree. Further, alfalfa cultivated with water containing additional mineral was remarkably improved in flavor and taste without lowering the relative extension rate in spite of containing mineral. The result reveals that cultivation of alfalfa using aqueous solution containing 1 w/v % of mineral and 1 w/v % of α,α-trehalose or α-maltosyl α,α-trehalose enables to improve the extension of aerial part of alfalfa and improve its flavor and taste.

### Experiment 9

### Removing fishy smell of freshwater fish

Two per group of living crucian *(Carassium cuvieri)* with body size in the range of 15 to 20 cm were bred at ambient temperature in an aquarium filled with 10 L of an aqueous solution containing 1 w/v % mineral (solar salt) and 1 w/v % α,α-trehalose or α-maltosyl α,α-trehalose, on a dry solid basis, for three days. Waters containing either of mineral, α,α-trehalose or α-maltosyl α,α-trehalose as references and water without any mineral and saccharides as a control were prepared to breed the fishes similarly as above. The fishes were macroscopically observed their activity, and then prepated into *sashimi.* Twenty panels were allowed to taste them to judge their flavor and taste (fishy smell) in such a manner of comparing with references by the following four judgment criterions; "good": no fishy smell; "slightly good": fishy smell is weaker than the references; "normal": fishy smell is equal to the references; and "bad": fishy smell is stronger than the references. The result is shown in Table 9.

**Table 9**

| Sample | Activity of *Carassius cuvieri* | Judgment of smell and taste | | | | Note |
|---|---|---|---|---|---|---|
| | | Good | Slightly good | Normal | Bad | |
| 1 (w/v)% mineral | Bad | 2 | 4 | 14 | 0 | Reference |
| 1 (w/v)% α,α-trehalose | Good | 3 | 10 | 7 | 0 | Reference |
| 1 (w/v)% α-maltosyl α,α-trehalose | Good | 2 | 12 | 6 | 0 | Reference |
| 1 (w/v)% mineral + 1 (w/v)% α,α-trehalose | Good | 17 | 3 | 0 | 0 | The present invention |
| 1 (w/v)% Mineral + 1 (w/v)% α-maltosyl α,α-trehalose | Good | 15 | 4 | 1 | 0 | The present invention |

As the result from Table 9, the fishes lost their activity when bred in the aqueous solution containing mineral at a concentration of 1 (w/v) %, and showed an insufficient improvement in flavor and taste such as fishy smell. In contrast, in the case of breeding with the above aqueous solution supplemented with α,α-trehalose or α-maltosyl α,α-trehalose, the fishes kept their activity and showed sufficient improvement in flavor and taste. While, sole use of α,α-trehalose or α-maltosyl α,α-trehalose showed insufficient improvement effect. The results revealed that aqueous solution containing mineral and α,α-trehalose or α-maltosyl α,α-trehalose exerts an effect of improving flavor and taste of crucian without loss of activity.

### Experiment 10

### Treatment for a disease of gold fish

One per group of gold fish (Japanese gold fish, *Carassius auratus*) with columnaris disease was bred in an aquarium filled with 2 L of aqueous solution containing 1 w/v % mineral and 1 w/v % α,α-trehalose or α-maltosyl α,α-trehalose at ambient temperature with normal feed for one week. Waters containing either of mineral, α,α-trehalose or α-maltosyl α,α-trehalose as references and water as a control were prepared to breed similarly as above. The fishes were macroscopically observed their activity and treatment effect. The result is shown in Table 10.

**Table 10**

| Sample | Activity of gold fish | Treatment effect | Note |
|---|---|---|---|
| Water | Good | Bad | Control |
| 1 (w/v)% mineral | Bad | Good | Reference |
| 1 (w/v)% α,α-trehalose | Good | Bad | Reference |
| 1 (w/v)% α-maltosyl α,α-trehalose | Good | Bad | Reference |
| 1 (w/v)% mineral + 1 (w/v)% α,α-trehalose | Good | Good | The present invention |
| 1 (w/v)% mineral + 1 (w/v)% α-maltosyl α,α-trehalose | Good | Good | The present invention |

As the result from Table 10, the gold fish bred in the aqueous solution containing mineral (sodium chloride) at a concentration of 1 (w/v) % showed a satisfactory effect on treating columnaris disease with loss of their activities. While, the gold fish bred in those further containing α,α-trehalose or α-maltosyl α,α-trehalose showed the equal treating effect without loss of their activities. In contrast, only α,α-trehalose or α-maltosyl α,α-trehalose was insufficiently effective on treating columnaris disease. These results revealed that aqueous solutions containing mineral (sodium chloride) and α,α-trehalose or α-maltosyl α,α-trehalose do not affect gold fishes even when bred in aqueous environment with an unsuitable mineral (sodium chloride) concentration of 1 w/v %.

The following examples explain the present invention in detail.

### Example 1

### Premixed powder of artificial seawater

A premixed powder of artificial seawater was produced according to the following formula. The product can be used as seawater after dissolved in 1,000 parts by weight of water. Since the product enhances biological activity and/or expands suitable region for biological activity of living bodies, it is used for reducing undesirable influences caused by the change of mineral concentration due to a metrical error, evaporation of moisture or influx of rain water, etc. Therefore, the product is advantageously used for breeding seawater fishes and shellfishes in fishpond or aquarium.

| | |
|---|---|
| Sodium chloride | 28.5 parts by weight |
| Magnesium sulfate, septahydrate | 6.82 parts by weight |
| Magnesium chloride, hexahydrate | 5.16 parts by weight |
| Calcium chloride, dihydrate | 1.47 parts by weight |
| Potassium chloride | 0.725 part by weight |
| Sodium bromide | 0.084 part by weight |
| Boric acid | 0.0273 part by weight |
| Strontium chloride hexahydrate | 0.024 part by weight |
| "TREHA®", a product name of a hydrous crystalline α,α-trehalose, commercialized by Hayashibara Shoji, Inc. | 20.0 parts by weight |

### Example 2

### Cleaning fluid

One part by weight of sodium chloride and four parts by weight of "HALLODEX", a product name of a liquid containing 52% of α-maltosyl α,α-trehalose, on a dry solid basis, commercialized by Hayashibara Shoji Inc., were dissolved in 100 parts by weight of distilled water to produce a cleaning fluid. Since the product enhances biological activity and/or expands suitable region for biological activity of freshwater fishes and shellfishes or agricultural products, it is advantageously used as a cleaning fluid for freshwater fishes and shellfishes including *Corbicula* and *Carassius* or fresh vegetables including sprout foods. In addition, the product is advantageously used as an agent of removing mud or deodorant for freshwater fishes and shellfishes.

### Example 3

### Composition for salt bath

One part by weight of commercialized powdery sodium chloride and one part by weight of "TREHA®", a product name of a hydrous crystalline α,α-trehalose, commercialized by Hayashibara Shoji Inc., were mixed well and admixed with appropriate antibacterial agents or antibiotics to produce a composition for salt bath. The product is applicable to salt-bathing for treating diseases of freshwater fishes such as a gold fish and *nishikigoi* (colored carp) when it is dissolved in an appropriate amount of water to give a sodium chloride concentration in the range of 0.5 to 2 w/v %. The composition for salt bath of the present invention can be directly incorporated in aquariums or ponds where the objective fishes are living because it also reduces undesirable influences caused by sodium chloride to waterweeds living with the fishes.

### Example 4

### Fertilizer stick

Fourteen parts by weight of a combination fertilizer (N = 14%, P₂O₅ = 8%, K₂O = 12%), three parts by weight of calcium sulfate, two parts by weight of magnesium chloride hexahydrate, one part by weight of pullulan, one part by weight of "TREHA®", a product name of a hydrous crystalline α,α-trehalose, commercialized by Hayashibara Shoji Inc., one part by weight of "HALLODEX", a product name of a syrup containing saccharide derivatives of α,α-trehalose, commercialized by Hayashibara Shoji Inc., and 0.5 part by weight of water were mixed well and subjected to an injection molding machine (L / D = 20, compression ratio = 1.8, bore diameter of die = 30 mm) at 80° C to produce a fertilizer stick. The product needs no special container and it can be used as a gradually-degradable fertilizer stick. It can be safely used without concerning a fertilizer burn because it reduces undesirable influences on plants even when the mineral concentration in transiently increased.

### Example 5

### Agent for enhancing biological activity and/or expanding suitable region for biological activity

Two parts by weight of "TREHA®", a product name of a hydrous crystalline α,α-trehalose, commercialized by Hayashibara Shoji Inc., was dissolved in 100 parts by weight of water to produce an agent for enhancing biological activity and/or expanding suitable region for biological activity. The product is capable of preventing salt damage for agricultural products by typhoon or tsunami when used as an agricultural water for tank farming or sprinkler after diluted, if necessary. In addition, the product enhances biological activity and/or expanding suitable region for biological activity of living bodies when added to seawater, culture medium or cleaning fluid containing minerals. It reduces undesirable influences caused by the change of mineral concentration due to the evaporation of moisture or the influx of rain water. Therefore, the product is advantageously used for breeding, cleaning and preserving organisms living in aqueous environment.

### Example 6

### Agent for enhancing biological activity and/or expanding suitable region for biological activity

Three parts by weight of a powder containing α-maltosyl α,α-trehalose (with a purity of 98 w/w% or more), prepared according to the method in Experiment 4 in Japanese Patent Kokai No.143876/1995, was dissolved in 100 parts by weight of water to produce an agent for enhancing biological activity and/or expanding suitable region for biological activity. The product prevents salt damage for agricultural products by typhoon or tsunami when used as agricultural water for tank farming or sprinkler after diluted, if necessary. In addition, the product enhances biological activity and/or expands suitable region for biological activity of living bodies when added to seawater, culture medium or cleaning fluid containing minerals. It reduces undesirable influences caused by the change of mineral concentration due to the evaporation of moisture or the influx of rain water. Therefore, the product is advantageously used for breeding, cleaning and preserving organisms living in aqueous environment.

### INDUSTRIAL APPLICABILITY

As explained above, the present invention enables to activate biological activity and enhance biological activity and/or expand suitable region for biological activity of living bodies when in mineral solutions. Since the present invention enhances biological activity and/or expands suitable region for biological activity of living bodies such as animals and plants including agricultural products and cultured fishes and shellfishes, the present invention is capable of reducing undesirable influences caused by the change of mineral concentration due to addition of excess amount of minerals, evaporation of moisture, or influx of rain water.

## Claims

1. A method for enhancing biological activity and/or expanding suitable region for biological activity of a living body contacting with aqueous environment, which comprises a step of allowing α,α-trehalose and/or a saccharide derivative thereof to coexist in said aqueous environment.

2. The method of claim 1, wherein said aqueous environment is an aqueous solution with a mineral concentration of 0 to 5 w/w % and said α,α-trehalose and/or said saccharide derivative thereof are in an amount of 0.01 to 4 w/v %.

3. The method of claim 1 or 2, wherein said aqueous environment has a mineral concentration higher or lower by ±2.0% in number in addition to the percentage of a mineral concentration suitable for biological activity of the living body.

4. The method of any one of claims 1 to 3, wherein said saccharide derivative thereof is a glycosyl α,α-trehalose.

5. The method of any one of claims 1 to 4, wherein said mineral is one or more members selected from the group consisting of sodium, magnesium, aluminum, potassium, calcium, chromium, manganese, iron, cobalt, nickel, copper, zinc, molybdenum and lead.

6. The method of any one of claims 1 to 5, which is used for reducing undesirable influence due to unsuitable mineral concentration, protecting agricultural products from salt damage, supplying minerals for agricultural products, breeding fishes and shellfishes, deodorizing fishes and shellfishes, removing sand from shellfishes, or treating diseases of freshwater fishes.

7. The method of any one of claims 1 to 6, wherein said living body is a saltwater or freshwater fish or shellfish.

8. A composition for enhancing biological activity and/or expanding suitable region for biological activity of a living body contacting with aqueous environment, which comprises a mineral in an amount of 0 to 5 w/v % and α,α-trehalose and/or saccharide derivative thereof contained in the aqueous environment in a total amount of 0.01 to 4 w/v % to the moisture content and/or to be contacted with the living body.

9. The composition of claim 8, wherein said aqueous environment has a mineral concentration higher or lower higher or lower by ±2.0% in number in addition to the percentage of a mineral concentration suitable for biological activity of the living body.

10. The composition of claim 8 or 9, wherein said mineral is one or more members selected from the group consisting of sodium, magnesium, aluminum, potassium, calcium, chromium, manganese, iron, cobalt, nickel, copper, zinc, molybdenum and lead.

11. The composition of any one of claims 8 to 10, which is a natural seawater, artificial seawater, washing, disinfectant, culture medium, or a premix or concentrated solution thereof.

12. An agent for enhancing biological activity and/or expanding suitable region for biological activity of a living body contacting with aqueous environment, which comprises α,α-trehalose and/or a saccharide derivative thereof as an effective ingredient.

13. The agent of claim 12, which is in the form of an aqueous solution containing α,α-trehalose and/or a saccharide derivative thereof in an amount of 0.01 to 4 w/v %.

14. A method for preventing weight loss of a living fish or shellfish, which comprises a step of breeding said fish or said shellfish in an aqueous solution containing α,α-trehalose and/or saccharide derivative thereof in an amount of 0.01 to 4 w/v %.

15. A method for increasing the content of succinic acid and/or malic acid, which comprises a step of breeding shellfish in an aqueous solution containing α,α-trehalose and/or saccharide derivative thereof in an amount of 0.01 to 4 w/v %.
